# EUROPEAN PATENT SPECIFICATION

(11) **EP 1 246 530 B1**
(45) Date of publication and mention of the grant of the patent: **10.08.2005**
(21) Application number: 01900861.4
(22) Date of filing: 04.01.2001
(51) Int. Cl.: A01N 25/34, A01N 59/16, A61K 33/24, A61K 33/38

(54) **HIGH PERFORMANCE SILVER (I, III) OXIDE AND COBALT (II, III) OXIDE ANTIMICROBIAL TEXTILE ARTICLES**
HOCHAKTIVE ANTIMIKROBIELLE TEXTILARTIKEL AUF DER BASIS VON SILBER(I,III)-OXID UND COBALT(II,III)-OXID
ARTICLES TEXTILES ANTIMICROBIENS A L'OXYDE D'ARGENT (I, III) ET A L'OXYDE DE COBALT (II, III) A HAUTE PERFORMANCE

(30) Priority: 05.01.2000 US 477883; 04.04.2000 US 542199
(43) Date of publication of application: 09.10.2002
(73) Proprietor: Marantech Holding, LLC, Providence, RI 02903 (US)
(72) Inventor: ANTELMAN, Marvin, S., Rehovot (IL)
(74) Representative: Winckels, Johannes Hubertus F.
(86) International application number: PCT/US2001/000213
(87) International publication number: WO 2001/049115

(56) References cited:
- US-A- 2 791 518
- US-A- 5 211 855
- US-A- 5 336 499
- M.S.ANTELMAN: "silver (II,III) disinfectants" SOAP, COSMETICS, CHEMICAL SPECIALTIES, vol. 70, no. 3, 1994, XP009002048

## Description

### FIELD OF THE INVENTION

This invention relates to textile articles possessing antimicrobial properties and a method for their preparation.

### DESCRIPTION OF RELATED ART

Chemical treatment of textile articles to render them microbicidal to microorganisms coming in contact with them is known in the art. Such articles include those made from paper, fibers, woven and non-woven textiles, and like fabrics which are designed for use in environments such as hospitals, food processing plants, laboratories, and other areas where maintenance of germ-free conditions is essential.

For example, U.S. Patent No. 2,791,518 to Stokes, Jr. et al. discloses a method of imparting microbicidal properties to articles such as textiles. The method recites to first immerse the article in a first aqueous solution containing a water-soluble basic nitrogen compound (e.g., ammonia) and a monovalent silver salt, soluble in the solution, followed by a second immersion in a second solution containing a second salt, capable of ion exchange with the silver salt, such that a monovalent silver salt precipitate is formed within the article. The formed silver precipitate is sparingly water-soluble and imparts microbicidal properties to the articles so treated.

Similarly, U.S. Patent No. 5,271,952 to Liang et al. discloses a method of treating fibers to render them electrically conductive as well as anti-bacterial. The method includes immersing the fibers in a bath containing an aqueous solution of a source of divalent copper ions, a reducing agent, sodium thiosulfate, and a source of iodide ions, whereby copper iodide is adsorbed into the fibers. Similar techniques for rendering fibers conductive or resistant to bacteria involving the use of copper compounds are disclosed in U.S. Patent Nos. 4,410,593 to Tomibe et al. and 5,458,906 to Liang.

It has also been disclosed that materials such as chlorinated hydantions may be grafted to textiles for the purpose of imparting antimicrobial properties. *See, e.g.,* Williams et al., 218th ACS National Meeting (1999) Abstracts, Cell 32; C&EN September 6, page 36. Textiles so treated tend to suffer severe diminishment of antimicrobial properties after as few as 5 hours of laundering and are unstable after long durations of exposure to ultraviolet light.

One of the main problems associated with the use of monovalent silver compounds, such as the halides, phosphates, or sulfates, in such applications is that they are sensitive to ultraviolet light and are thus prone to discoloration after exposure to sunlight, with a gradual loss of effectiveness of antimicrobial properties. Also, many of these compounds are soluble or slightly soluble in hot water which diminishes the antimicrobial properties after only a few launderings of reusable fabrics containing them.

Another problem with respect to the use of copper compounds as interstitially precipitated antimicrobials, described in U.S. Patent Nos. 5,271,952 and 5,458,906, is that these processes do not readily lend themselves to inclusion in textile production lines. Textile finishing lines involve processes which take minutes and cannot readily accommodate precipitations which require lengthy immersion times of sixty minutes or more.

The main object of the present invention is to provide a method of treating fibers and configurated textile products so that anti-microbial properties are imparted to them. Another object of the invention is to incorporate into the fibers and textile products powerful anti-microbial properties based on an electron active molecular crystal analogous to the molecular crystal, tetrasilver tetroxide, which has been proven to be one of the most powerful disinfectants known to man. Another object of the invention is to form a multivalent cobalt oxide by interstitial precipitation. Still another object of the invention is to enable rapid mass production of such anti-microbial fibers and textile products.

### SUMMARY OF THE INVENTION

The invention relates to a fibrous textile article including a silver (I, III) or cobalt (II, III) oxide as an antimicrobial agent interstitially deposited within the article, the agent present in an amount sufficient to impart antimicrobial properties to the article. In the embodiment with cobalt (II, III) oxide, the article may also include a source of fluoride ions.

In one embodiment, the antimicrobial agent is interstitially deposited by interstitial precipitation. The article can include woven or non-woven fabric, or both. In a preferred embodiment, the antimicrobial agent is present within the fabric at a level of about 0.5 to about 15,000 weight PPM, based on the weight of silver or cobalt. The antimicrobial properties are sufficient to yield microbial inhibition zones extending beyond 1 mm of fabric swatch borders as measured by AOAC test 972.04.

The invention further relates to a process for imparting antimicrobial properties to a fibrous textile article, including providing a first aqueous solution including a water-soluble silver or cobalt salt; contacting the article with the first aqueous solution for a period of time sufficient to uniformly wet the article with the first aqueous solution; immersing the wetted article in a bath including a second aqueous solution including a strong alkali and a water soluble oxidizing agent and heating the bath for a period of time sufficient to interstitially precipitate tetrasilver tetroxide or cobalt (II, III) oxide with the article to yield a finished article; and removing the finished article from the bath.

In preferred embodiments, the silver salt includes silver nitrate or the cobalt salt includes cobalt chloride. The bath for the cobalt salt can further include a water-soluble fluoride salt, preferably at a concentration of about 10 to 1500 mg/L. In preferred embodiments, the alkali includes sodium hydroxide or potassium hydroxide and the oxidizing agent is sodium persulfate or potassium persulfate.

In one embodiment, the finished article includes about 0.5 to 15,000 PPM of the salt, based on the weight of the article. In preferred embodiments, the contact of the article with the first aqueous solution includes immersion of the article in the first aqueous solution for about 15 seconds to about 60 seconds and the time of immersion of the article in the second aqueous solution is about 30 seconds to about four minutes.

### DETAILED DESCRIPTION OF THE INVENTION

Imparting antimicrobial properties to fiber and textile products is achieved in the instant invention by interstitial deposition of the molecular crystal compound tetrasilver tetroxide, *i*.*e*., silver (I, III) oxide, or multivalent cobalt oxide, *i*.*e*., cobalt (II, III) oxide. The silver moiety is the subject of several patents. U.S. Patent No. 5,336,499 to Antelman, the disclosure of which is incorporated herein by reference, describes the anti-pathogenic properties of silver oxide of formula Ag₄O₄ and also the mechanism of operation of the molecular device, based on a unique crystal having two monovalent silver (Ag I) ions and two trivalent silver (Ag III) ions in the molecule. It has now been found that cobalt (II, III) oxide (Co₃O₄) is capable of killing pathogens in a like manner in textile articles.

U.S. Patent No. 5,211,855 to Antelman also discloses the use of Ag₄O₄ crystals to kill pathogens in utilitarian water bodies such as swimming pools.

An antimicrobial spectrum of Ag₄O₄ shown in Table 1 can be found in Antelman, "Silver (II, III) Disinfectants," *Soap Cosmetics Chemical Specialties* 1994, 70, 3 pp. 52-59. The spectrum is based on specifications of the Association of Official Analytical Chemists ("AOAC")

**Table 1.**

| **Antimicrobial Spectrum of Ag**_{**4**}**O**_{**4**} | |
|---|---|
| MICROORGANISM | MIC* (ppm) |
| **Gram Negatives** | |
| Escherichia coll 10231 | 2.50 |
| Escherichia coll 25254 | 2.50 |
| Enterobacter cloacae 13047 | 1.25-2.50 |
| Pseudomonas aeruginosa 9027 | 2.50 |

| **Gram Positives** | |
|---|---|
| Bacillus subtilis 6633 | 1.25-2.50 |
| Micrococcus lutena 9341 | 2.50-5.00 |
| Staphylococcus aureus 0927 | 5.00 |
| Staphylococcus aureus 27543 | 0.625 |
| Staphylococcus epidermidis 12228 | 1.25-5.00 |
| Streptococcus agalactiae 27956 | 5.00 |
| Streptococcus faecium 10541 | 2.50 |
| Streptococcus pyogenes 7958 | 5.00 |

| **Yeast and Mold** | |
|---|---|
| Candida albicans 16404 | 2.50-5.0 |
| Saccharomyces cerevisiae 2601 | 1.25 |

| | |
|---|---|
| * minimal inhibitory concentration | |

Monovalent silver is more anti-pathogenic than mercury which is more anti-pathogenic than copper, based on their oligodynamic activity as articulated by J.G. Horsfal in "Principles of Fungicidal Action" (Chronica Botanica 1956). The relative efficacy of metallic moieties against pathogens has been called the Horsfal series:

Ag>Hg>Cu>Cd>Cr>Ni>Co>Zn>Fe>Ca

It has been found that with respect to a Horsfal series dedicated to silver, the relative efficacy against pathogens is as follows:

Ag₄O₄> Ag(III) > Ag(III) > Ag(I)

The term "fibrous textile article" as used herein is intended to encompass a wide variety of materials including paper, natural or synthetic fibers, threads, and yarns made from materials such as cotton, rayon, wool, jute, nylon, polyesters, polyacetates, polyacrylics, as well as cellulosics in general. More particularly, the term refers to fibers woven into a fabric such as knitting, and non-woven hydrophilic fabrics or webbing used in anti-pathogenic applications such as in the medical field, hospitals, biotechnology, and food and dairy processing. Examples of textile products in these fields include bandages, gauze, bandage pads, skin patches, work clothes (both disposable and reusable), bed sheets, masks, dust cloths, safety belts, surgical gowns, ambulance blankets, stretchers, filter materials, diapers, underwear, pajamas, video display terminal screens, and the like.

For some antimicrobial applications, a silver oxide or cobalt oxide, such as Ag₄O₄ or Co₃O₄ crystals, or both, may be deposited within the interstices of fibrous articles by simply soaking the article in an aqueous dispersion of the crystals (generally having a dimension of less than 100 angstroms), or by combining the crystals with a carrier medium and applying this composition to the fibrous article. This method of physical incorporation of the crystals is useful where the article is composed of low density or loosely associated fibers, such as bandage pads, gauze pads, and loosely non- woven products, and particularly laminated products wherein the treated fibrous article is subsequently sandwiched between one or two peelable layers which tend to keep the crystals trapped in the fibrous article until ready for use. Also, antimicrobial paper products may be made by simply mixing an aqueous dispersion of the oxide, such as Ag₄O₄ or Co₃O₄ crystals, or both, with paper pulp prior to calendaring the pulp.

Physical incorporation of the crystals may tend to be less effective, however, where the treated article is a fiber or yam or a higher density woven or non-woven fabric, since the pre-formed crystals can not penetrate the interstices of such articles as easily. Deposition of the silver oxide or cobalt oxide material via interstitial precipitation is preferred in such cases.

Interstitial precipitation of the metal oxide crystals (generally having an average dimension of less than 100 angstroms) is accomplished by first providing an aqueous solution of, in the case of a silver oxide, a monovalent water-soluble silver salt such as the nitrate, perchlorate, acetate, methanesulfonate, or fluoride, most preferably silver nitrate. Next, the article to be treated, *e*.*g*., a fiber or yam of a woven or non-woven fabric, is thoroughly wetted with this solution such that the article absorbs the solution on fiber surfaces as well as one or more of the interstices between the fibrils forming the fiber, between fibers forming the yam or non-woven fabric, or between the weft and woof yarns present in woven fabrics. Wetting may be accomplished by uniformly spraying the article, or more preferably by dipping the article in a bath of the silver or cobalt salt solution for a period of time sufficient for the article to absorb the requisite amount of silver or cobalt salt solution. This time may range from about 15 seconds to 60 seconds, more preferably about 30 seconds.

Next, the wetted article is removed from the immersion bath, optionally squeezed to remove excess solution, and immersed in a heated bath containing a second aqueous solution containing a strong alkali and a water soluble oxidizing agent, and heated for a period of time sufficient to cause reaction leading to the interstitial precipitation of tetrasilver tetroxide (Ag₄O₄) or Co₃O₄ crystal material in the interstices of the fibrous article. Suitable alkalis for this purpose include sodium or potassium hydroxide, with sodium hydroxide most preferred. Suitable oxidizing agents include alkali metal persulfates. Sodium, or more preferably potassium persulfate, is the preferred oxidizer. Reaction of the Ag₄O₄ in the bath is accomplished by heating at a temperature of at least about 85 °C, more preferably at least about 90 °C, for a period of time sufficient to maximize yield of Ag₄O₄, generally from about 30 seconds to about 5 minutes. Reaction for the Co₃O₄ in the bath is accomplished at room temperature.

After the reaction is completed, the treated article is removed from the bath and may be washed several times with water to remove soluble inpurities or unreacted reagant.

The quantity of Ag₄O₄ or Co₃O₄ material present in the resulting article will generally be a function of the quantity of silver or cobalt salt sorbed by the article, which can vary depending on the nature of the article, *e.g.*, loose vs. tight weave fabrics or whether the fiber is natural or synthetic, the former being more absorbant of the silver or cobalt salt solution.

For the tetrasilver tetroxide, in general, the quantity of alkali present in the second bath is preferably sufficient to maintain a strongly basic pH, *i*.*e*., greater than about 13, and provide a slight molar excess of silver salt over oxidizing agent, suitable to complete the reaction. Thus, the content of tetrasilver tetroxide interstitially precipitated within any given fibrous article may be controlled by varying the concentration of the silver salt in the solution used to first wet the article and appropriately adjusting the quantities of alkali and oxidizing agent present in the immersion solution at approximately stoichiometric levels.

For the Co₃O₄, in general, the quantity of alkali present in the second bath should be sufficient to maintain a pH on the basic side, i.e., above about 9. The content of Co₃O₄ crystals interstitially precipitated within any given fibrous article may be controlled by varying the concentration of the cobalt salt in the solution used to first wet the article.

The term "derivatives of Ag₄O₄" is intended to include Ag₄O₄ reaction products prepared by reacting Ag₄O₄ with suitable water-soluble acids to give the corresponding Ag (II) salts, e.g., reactions with fluoroboric acid or phosphoric acid to give the Ag (II) fluoroborate or phosphate, as disclosed in U.S. Patent No. 5,107,295 to Ibuhci. Divalent silver nitrate and divalent silver halides prepared by reacting Ag₄O₄with nitric acid or the corresponding haloacids, e.g., HBr, HI or HCl as disclosed in U.S. Patent No. 5,078,902 to Antelman are also included. Trivalent silver derivatives such as Ag (III) biguanide prepared in accordance with U.S. Patent No. 5,223,149 to Antelman are also included.

Textile articles containing such derivatives are prepared by further contacting the Ag₄O₄-containing article in an additional step with an aqueous solution containing up to stoichiometric amounts of the appropriate reagant(s) sufficient to convert at least a portion of the Ag₄O₄ to the Ag (II) or Ag (III) derivative.

The actual tetrasilver tetroxide is more preferred than textile articles containing such derivatives because some derivatives may be generally more water-soluble than Ag₄O₄, such that they can require a further processing step in their manufacture and can be less effective as antimicrobial agents than Ag₄O₄ as shown in the silver Horsfal series described above. Ag(II) or Ag(III) derivatives of Ag₄O₄ are, however, useful as antimicrobial agents in fabrics designed for a single use, such as bandages or disposable garments.

The content of the Ag₄O₄ or its derivatives (based on weight PPM silver) in the fabric may preferably range from as little as 0.5 weight PPM up to about 50,000 weight PPM, based on the weight of the textile article. The minimum content should be sufficient to kill pathogens from which protection is sought, whereas the maximum content is dictated by factors such as economy and affect on fabric properties. Generally, the higher the silver content, the more effective will be the antimicrobial properties of the article. Silver content in the range of from about 30 to about 10,000 weight PPM will provide satisfactory antimicrobial properties for most applications.

The antimicrobial properties of the articles treated with Co₃O₄ may be further enhanced by including a source of fluoride ions in the second oxidizing bath described above. Such sources include water-soluble fluoride salts, such as sodium or potassium fluoride. The amount of fluoride anion source may generally range from about 10 mg to 1500 mg per liter of solution, preferably from about 100 mg to 1000 mg per liter.

The content of the Co₃O₄(based on weight PPM cobalt) in the fabric may range from as little as about 0.5 weight PPM up to about 15,000 weight PPM, based on the weight of the textile article. The minimum content should be sufficient to kill pathogens from which protection is sought, whereas the maximum content is dictated by factors such as economy and affect on fabric properties. Generally, the higher the cobalt content, the more effective will be the antimicrobial properties of the fabric. Cobalt content from about 1000 weight PPM to about 15,000 weight PPM will provide satisfactory antimicrobial properties for most applications.

Antimicrobial properties are evaluated in accordance with this invention using the AOAC test method 972.04, which is used primarily to evaluate the bacteriostatic activity of laundry additive disinfectants. In this test, a square or rectangular sterile swatch of fabric is pressed into a petri dish containing a layer of nutrient agar that has been inoculated with a pathogen. Following a fixed period of incubation, each fabric sample is evaluated by measuring the clear zones adjacent the four sides of each test swatch as an index of antimicrobial activity. The presence of clear zones along all four sides of the swatch is indicative of antimicrobial activity, rated 4/4. The width of the clear zones in millimeters is reasonably indicative of the degree of antimicrobial activity.

### EXAMPLES

The invention is further defined by reference to the following examples describing the invention in detail.

### Example 1: A Nylon Article Prepared According to the Invention

A swatch of virgin nylon webbing was immersed in an aqueous solution containing dissolved silver nitrate at a concentration of about 100 PPM silver maintained at room temperature. After 30 seconds immersion time, the swatch was removed from this solution and immersed in a hot aqueous solution containing 7.2 g/liter each of NaOH and sodium persulfate. The solution was then boiled for one minute (95-100°C). The swatch was then removed from the boiling solution, washed with water, and dried.

A barely visible tan coating was observed on the swatch fibers. The content of Ag₄O₄ in the fabric swatch, measured as silver, was 89 weight PPM as verified by gravimetric analysis.

### Examples 2-6: Nylon Articles Prepared According to the Invention

Example 1 was repeated, except that the concentration of silver nitrate in the first solution and reagents in the second solution were varied to provide the following Ag content in the fabric swatch:

| SOLUTION (PPM Ag) | | FABRIC (PPM Ag) |
|---|---|---|
| Ex 2 | 890 | 35* |
| Ex 3 | 890 | 541 |
| Ex 4 | 10,000 | 3970 |
| Ex 5 | 10,000 | 9140 |
| Ex 6 | 10,000 | 9140 |

| | | |
|---|---|---|
| *Nylon fabric for this test was of a tighter weave than that used in Example 1, which accounts for the lower silver absorption. | | |

AOAC antipathogenic tests on these textiles were performed by an independent laboratory licensed by a State environmental regulatory body. The marker organisms used in conformity with AOAC test method 972.04 were Pseudemonas Aeroginosa (PA) as the Gram negative bacteria marker, and Staphylococcus Aureus (SA) for Gram positive bacteria.

The tests were conducted in terms of inhibition of cultures of the bacteria. Two swatches were used for the tests in contact with the cultures. The swatches were 1.5 inches wide and had a density of about 69 mg/cm². Each swatch had four sides, and two swatches were used with each representative culture so that a total of eight trials were reflected with each bacterium. An 8/8 inhibition would indicate 100% efficacy. The test protocol, however, went beyond the specifications of the AOAC method in that the actual average inhibition zone width in millimeters was recorded for both swatches tested. These results were then combined with the anti-microbial spectrum shown in Table 1 which includes the marker bacteria of the AOAC tests, and extrapolated. The conclusion was that the preferred embodiments of the invention were 100% effective against all of the microbes shown in Table 1 and against salmonella based on the previous independent results obtained with silver (I, III) oxide.

Representative results with nylon fabric are shown in Table 2. Generally, the degree of microbial activity varies directly with the silver concentration. In addition to the high performance anti-microbial properties of the fabrics, they withstood wear and could be considered permanent in that tested fabrics withstood 100 hours of laundering and 600 hours of ultraviolet light exposure. Laundering is evaluated using hot water and detergent using the standard test of the American Association of Textile Chemists ("AATC")

**Table 2.**

| **Antimicrobial Performance of Precipitated Ag**_{**4**}**O**_{**4**} | | | | |
|---|---|---|---|---|
| Example | Silver (ppm) | Inhibition Zone-SA (mm) | Inhibition Zone-PA (mm) | Inhibition Index |
| 1 | 89 | 3.2 | 1.3 | 8/8 |
| 2 | 35 | 1.8 | 2.0 | 8/8 |
| 3 | 541 | 5.5 | 5.1 | 8/8 |
| 4 | 3970 | 5.8 | 2.6 | 8/8 |
| 5 | 9140 | 5.8 | 2.8 | 8/8 |
| 6 | 9670 | 6.1 | 4.8 | 8/8 |

### Example 7: Efficacy of Textile Articles of the Invention Against Staph Pathogen

An independent medical researcher in Israel obtained a very virulent strain of Staph from a patient at the Shaarei Tzedek Hospital in Jerusalem. The patient subsequently died from infection. This strain was evaluated as more virulent than any of the other Staph microorganisms listed in Table 1 by the pathology staff at the hospital. This Staph strain was utilized as the Staph source, and the otherwise exact test protocol described above was repeated. The silver concentration of the test swatch was found to be 9,138 PPM. Only one test swatch was used for the Staph evaluation. It tested at 4/4 with a much diminished average inhibition zone of 0.50 mm, which was to be expected for the more virulent strain. The values were extrapolated for all Gram positive bacteria listed in Table 1. It was concluded that precipitated Ag₄O₄ was capable of inhibiting all of the listed Gram positive bacteria. The extrapolation takes into consideration a theoretical calculation of the reduction of the Staph inhibition zone, were the conventional Staph aureus organisms to display the listed MIC range of 2.5 PPM to 5.0 PPM. Since the inhibition zone is inversely proportional to the MIC, one can calculate that the MIC for the virulent Staph strain was 30.5 PPM to 61.0 PPM. By applying the same reasoning to the Gram negative microorganisms for their PA marker, one can claim inhibition as well for all Gram negative bacteria listed in Table 1 by Ag₄O₄.

### Example 8: Efficacy of Nylon Articles of the Invention Against Staph Pathogen

Example 1 was repeated with larger amounts of webbing utilizing one foot lengths. Accordingly, webbing was obtained having 4,730 and 9,430 PPM of silver. These materials were dyed orange, and the dye completely covered and hid the brown/black color imparted to the virgin webbing by the tetrasilver tetroxide at these relatively high concentrations. After dyeing, swatches of the webbing were cut from the master rolls and were then evaluated in the same manner as described above by exposure to Staph aureus. All swatches indicated an 8/8 score, with average inhibition zones of 6.3 and 6.0 for the 4730 and 9430 PPM samples, respectively. Lengths of the dyed webbing were subjected to 100 hours of laundering in accordance with the AATC method, after which bacteriostatic efficacy was again evaluated. Visual inspection after laundering revealed frayed webbing. Nevertheless, both materials exhibited an 8/8 score with an improvement in inhibition zones to 7.0 for Staph aureus. This indicated that the laundry wear tended to expose fresh surface of tetrasilver tetroxide from the fabric interstices. Swatches were again taken from these materials and exposed to 600 hours of ultraviolet light in a weathering test. Evaluations of the UV exposed samples with Staph aureus again indicated scores of 8/8 for both concentrations of silver, with inhibition zones of 6.5 and 4.6, for the 4730 PPM and 9430 PPM silver concentration webbing, respectively. The conclusion was that ultraviolet exposure did not interfere with bacteriostatic activity.

### Comparative Example

Monovalent silver iodide was interstitially precipitated within nylon fabric such that the fabric contained 4895 PPM silver. Test swatches were prepared and evaluated against SA and PA pathogens by the test procedure described above. The Inhibition Index for SA was 7/8 and for PA was 0/8. In addition, the SA inhibition zone was only about 0.5 mm.

The following tests were conducted in terms of inhibition of cultures of bacteria. Swatches were used for the tests in contact with the cultures, each swatch having four sides and two swatches being used with each representative culture so that a total of eight trials were reflected with each bacterium. An 8/8 inhibition would indicate 100% efficacy. The test protocol went beyond the specifications of the AOAC methods, however, in that the actual average inhibition zone in millimeters was recorded for both swatches tested. Accordingly, 8/8 positive results were obtained with the marker bacteria cultures. These results were then combined with the anti-microbial spectrum shown in Table 1 which includes the marker bacteria of the AOAC tests, and extrapolated. The conclusion was that the preferred embodiments of the instant invention were 100% effective against the listed Table 1 microbes.

### Example 9: Nylon Article Prepared According to the Invention

A swatch of virgin nylon webbing of a standard size, 2.0 mm by 5.0 mm, was taken and immersed for 30 seconds in a cobalt chloride solution containing 10,850 PPM cobalt. A gravimetric determination of the cobalt absorbed after immersion showed cobalt at 9,363 PPM in the webbing. The webbing was then immersed in a room temperature solution containing 50.0 grams/liter potassium persulfate and 22.5 grams/liter sodium hydroxide for two minutes. The webbing was then removed, washed, and dried. The fibers then had a dark brown coating. The swatch was divided into thirds to give three swatches, two for testing in compliance with the AOAC bacterial test 972.04. Zones of inhibition were obtained on all eight sides, *i.e.,* 8/8, for both marker organisms. The average inhibition zones were 1.0 mm for pseudomonas aeruginosa and 1.1 mm for Staph aureus.

### Example 10: Nylon Article Prepared According to the Invention

Example 9 was repeated in all aspects except that the solution chemistries were changed and the immersion time in the oxidizer was reduced to one minute. The cobalt as cobalt chloride was 5,400 PPM cobalt and on the fabric was 4,222 PPM. The persulfate oxidizer was identical to Example 9 except that 600 mg/liter of sodium fluoride was added. The results for the Staph marker bacteria were 8/8. The average inhibition zones were 2.0 mm. The addition of fluoride enhanced the efficacy of the fibers against Staph as seen in the inhibition zone being higher than in Example 9 with only 45% of the cobalt concentration that was effective in Example 9.

### Example 11: Efficacy of Nylon Article Prepared According to the Invention Against Staph

An independent medical researcher in Israel obtained a very virulent strain of Staph from a patient at the Shaarei Tzedek Hospital in Jerusalem. The patient subsequently died from the infection. This strain was evaluated as more virulent than any of the other Staph micro-organisms listed in Table 1 by the pathology staff at the Jerusalem hospital. This Staph strain was utilized as the Staph source by the researcher who performed the AOAC test 972.04 for bacterial retardation efficacy on the third swatch set aside in Example 9. Since there was only one swatch, there were only four sides to be tested. The virulent strain of Staph was inhibited 100% giving a reading of 4/4. The average inhibition for the 4/4 result was 1.5 mm. Since the virulent strain of Staph qualified on a silver (I, III) oxide scale as having an MIC of 30.5-61 PPM, the values were extrapolated for all Gram positive bacteria listed in Table 1. It was concluded that precipitated cobalt (II, III) oxide was capable of inhibiting all of the listed Gram positive bacteria. By applying the same reasoning to the Gram negative microorganisms of the Example 9 PA marker, one can claim inhibition as well for all Gram negative bacteria listed in Table 1.

Fabrics treated in accordance with this invention hold promise for many antimicrobial applications ranging from preventing jock itch when applied to athletic supporters to preventing scabies and bed sores with treated bed sheets or hospital gowns used in nursing homes and hospitals.

## Claims

1. A fibrous article that includes an antimicrobial agent interstitially deposited within the article, the agent comprising a silver (I, III) oxide or a cobalt (II, III) oxide and being present in the article in an amount sufficient to impart antimicrobial properties to the article.

2. The article of claim 1, wherein the antimicrobial agent is interstitially deposited by interstitial precipitation and the antimicrobial properties are sufficient to yield microbial inhibition zones extending beyond 1 mm of fabric swatch borders as measured by AOAC test 972.04.

3. The article of claim 1, wherein the textile includes woven or non-woven fabric and wherein the antimicrobial agent is present within the fabric at a level in the range of about 0.5 to about 15,000 weight PPM, based on the weight of the silver or cobalt.

4. The article of claim 3, wherein the fabric is capable of withstanding at least 100 hours of laundering without significant loss of antimicrobial efficacy.

5. The article of claim 3, wherein the fabric is capable of withstanding at least 600 hours exposure to ultraviolet light without significant loss of antimicrobial efficacy.

6. The article of claim 1, wherein the antimicrobial agent comprises a silver (I, III) oxide.

7. The article of claim 6, wherein the antimicrobial agent is selected from the group consisting of tetrasilver tetroxide and derivatives thereof.

8. The article of claim 7, wherein the antimicrobial agent is tetrasilver tetroxide.

9. The article of claim 1, wherein the antimicrobial agent comprises a cobalt (II, III) oxide.

10. The article of claim 9 further comprising a plurality of fluoride ions.

11. A process for imparting antimicrobial properties to a fibrous textile article comprising:
providing a first aqueous solution comprising a water-soluble silver or cobalt salt;
contacting the article with the first aqueous solution for a period of time sufficient to uniformly wet the article with the first aqueous solution;
immersing the wetted article in a bath comprising a second aqueous solution comprising a strong alkali and a water soluble oxidizing agent and heating the bath for a period of time sufficient to interstitially precipitate a silver (I, III) oxide or a cobalt (II, III) oxide within the article to yield a finished article; and
removing the finished article from the bath.

12. The process of claim 11, wherein the alkali is sodium hydroxide or potassium hydroxide.

13. The process of claim 11, wherein the oxidizing agent is sodium persulfate or potassium persulfate.

14. The process of claim 11, wherein the article comprises about 0.5 to about 15,000 PPM of the salt, based on the weight of the article.

15. The process of claim 11, wherein the contact of the article with the first aqueous solution comprises immersion of the article in the first aqueous solution for about 15 seconds to about 60 seconds.

16. The process of claim 11, wherein the time of immersion of the article in the second aqueous solution is about 30 seconds to about four minutes.

17. The process of claim 11, wherein the water-soluble silver salt is silver nitrate.

18. The process of claim 11, wherein the water-soluble cobalt salt is cobalt chloride.

19. The process of claim 18, wherein the bath further comprises a water-soluble fluoride salt.

20. The process of claim 19, wherein the concentration of fluoride salt in the bath is about 10 to 1500 mg/L.

21. The process of claim 11, wherein the second solution comprises an amount of alkali sufficient to provide a pH of at least about 13.

22. The process of claim 11, wherein the bath is heated to a temperature of at least about 85°C.

23. A process for imparting antimicrobial properties to a fibrous article comprising soaking the article in an aqueous dispersion of tetrasilver tetroxide crystals.

## Patentansprüche

1. Faserartikel, der ein antimikrobielles Mittel enthält, das interstitiell innerhalb des Artikels abgelagert ist, wobei das Agens ein Silber(I,III)oxid oder ein Cobalt(II,III)oxid umfasst und in dem Artikel in einer ausreichenden Menge vorhanden ist, um dem Artikel antimikrobielle Eigenschaften zu verleihen.

2. Artikel gemäß Anspruch 1, wobei das antimikrobielle Mittel durch interstitielle Fällung interstitiell abgelagert ist und die antimikrobiellen Eigenschaften ausreichen, um Zonen der mikrobiellen Hemmung zu ergeben, die sich gemäß einer Messung durch den AOAC-Test 972.04 bis jenseits von 1 mm der Stoffmustergrenzen des Textilstoffs erstrecken.

3. Artikel gemäß Anspruch 1, wobei das Textil ein Gewebe oder einen Vliesstoff umfasst und wobei das antimikrobielle Mittel innerhalb des Textilstoffs in einer Menge im Bereich von etwa 0,5 bis etwa 15 000 ppm (w/w) vorhanden ist, bezogen auf das Gewicht des Silbers oder Cobalts.

4. Artikel gemäß Anspruch 3, wobei der Textilstoff wenigstens 100 Stunden lang gewaschen werden kann, ohne seine antimikrobielle Wirksamkeit in erheblichem Ausmaß zu verlieren.

5. Artikel gemäß Anspruch 3, wobei der Textilstoff wenigstens 600 Stunden lang ultraviolettem Licht ausgesetzt werden kann, ohne seine antimikrobielle Wirksamkeit in erheblichem Ausmaß zu verlieren.

6. Artikel gemäß Anspruch 1, wobei das antimikrobielle Mittel ein Silber(I,III)oxid umfasst.

7. Artikel gemäß Anspruch 6, wobei das antimikrobielle Mittel aus der Gruppe ausgewählt ist, die aus Tetrasilbertetroxid und Derivaten davon besteht.

8. Artikel gemäß Anspruch 7, wobei es sich bei dem antimikrobiellen Mittel um Tetrasilbertetroxid handelt.

9. Artikel gemäß Anspruch 1, wobei das antimikrobielle Mittel ein Cobalt(II,III)oxid umfasst.

10. Artikel gemäß Anspruch 9, der weiterhin eine Menge von Fluorid-Ionen umfasst.

11. Verfahren, um einem Fasertextilartikel antimikrobielle Eigenschaften zu verleihen, umfassend:
Bereitstellen einer ersten wässrigen Lösung, die ein wasserlösliches Silber- oder Cobaltsalz umfasst;
In-Kontakt-Bringen des Artikels mit der ersten wässrigen Lösung während einer ausreichenden Zeitspanne, um den Artikel gleichmäßig mit der ersten wässrigen Lösung zu benetzen;
Eintauchen des benetzten Artikels in ein Bad, das eine zweite wässrige Lösung umfasst, welche ein starkes Alkali und ein wasserlösliches Oxidationsmittel umfasst; und
Erhitzen des Bades während einer ausreichenden Zeitspanne, um ein Silber(I,III)oxid oder ein Cobalt(II,III)oxid interstitiell innerhalb des Artikels auszufällen, wobei man einen fertigen Artikel erhält; und
Entnehmen des fertigen Artikels aus dem Bad.

12. Verfahren gemäß Anspruch 11, wobei es sich bei dem Alkali um Natriumhydroxid oder Kaliumhydroxid handelt.

13. Verfahren gemäß Anspruch 11, wobei es sich bei dem Oxidationsmittel um Natriumpersulfat oder Kaliumpersulfat handelt.

14. Verfahren gemäß Anspruch 11, wobei der Artikel etwa 0,5 bis etwa 15 000 ppm des Salzes umfasst, bezogen auf das Gewicht des Artikels.

15. Verfahren gemäß Anspruch 11, wobei der Kontakt des Artikels mit der ersten wässrigen Lösung das Eintauchen des Artikels in die erste wässrige Lösung während etwa 15 Sekunden bis etwa 60 Sekunden umfasst.

16. Verfahren gemäß Anspruch 11, wobei die Dauer des Eintauchens des Artikels in die zweite wässrige Lösung etwa 30 Sekunden bis etwa vier Minuten beträgt.

17. Verfahren gemäß Anspruch 11, wobei es sich bei dem wasserlöslichen Silbersalz um Silbernitrat handelt.

18. Verfahren gemäß Anspruch 11, wobei es sich bei dem wasserlöslichen Cobaltsalz um Cobaltchlorid handelt.

19. Verfahren gemäß Anspruch 18, wobei das Bad weiterhin ein wasserlösliches Fluoridsalz umfasst.

20. Verfahren gemäß Anspruch 19, wobei die Konzentration des Fluoridsalzes in dem Bad etwa 10 bis 1500 mg/l beträgt.

21. Verfahren gemäß Anspruch 11, wobei die zweite Lösung eine ausreichende Menge an Alkali umfasst, um einen pH-Wert von wenigstens etwa 13 zu erhalten.

22. Verfahren gemäß Anspruch 11, wobei das Bad auf eine Temperatur von wenigstens etwa 85 °C erhitzt wird.

23. Verfahren, um einem Faserartikel antimikrobielle Eigenschaften zu verleihen, umfassend das Tränken des Artikels in einer wässrigen Dispersion von Tetrasilbertetroxidkristallen.

## Revendications

1. Un article fibreux qui renferme un agent antimicrobien disposé interstitiellement dans l'article, l'agent comprenant un oxyde d'argent (I, III) ou un oxyde de cobalt (II, III) et étant présent dans l'article en une quantité suffisante pour lui conférer des propriétés antimicrobiennes.

2. L'article selon la revendication 1, dans lequel l'agent antimicrobien est déposé interstitiellement par précipitation interstitielle et dans lequel les propriétés antimicrobiennes sont suffisantes pour produire des zones d'inhibition microbiennes débordant de plus de 1 mm les liserés du coupon, tel que mesuré par la norme AOAC 972.04.

3. L'article selon la revendication 1, dans lequel le textile comprend des tissus tissés ou non tissés et dans lequel l'agent antimicrobien est présent dans le tissu en une proportion comprise entre environ 0,5 et environ 15 000 ppm en poids, exprimés par rapport au poids d'argent ou de cobalt.

4. L'article selon la revendication 3, dans lequel le tissu peut supporter au moins 100 heures de lessive sans perte notable de l'efficacité antimicrobienne.

5. L'article selon la revendication 3, dans lequel le tissu peut supporter au moins 600 heures d'exposition à la lumière ultraviolette sans perte notable de l'efficacité antimicrobienne.

6. L'article selon la revendication 1, dans lequel l'agent antimicrobien comprend un oxyde d'argent (I, III).

7. L'article selon la revendication 6, dans lequel l'agent antimicrobien est choisi dans le groupe consistant en tétroxyde de tétraargent et ses dérivés.

8. L'article selon la revendication 7, dans lequel l'agent antimicrobien est le tétroxyde de tétraargent.

9. L'article selon la revendication 1, dans lequel l'agent antimicrobien comprend un oxyde de cobalt (II, III).

10. L'article selon la revendication 9, comprenant en outre une pluralité d'ions fluorure.

11. Un procédé pour conférer des propriétés antimicrobiennes à un article textile fibreux comprenant:
- l'emploi d'une première solution aqueuse comprenant un sel hydrosoluble d'argent ou de cobalt;
- le contact de l'article avec la première solution aqueuse pendant une durée suffisante pour humidifier uniformément l'article par la première solution aqueuse;
- l'immersion de l'article mouillé dans un bain comprenant une seconde solution aqueuse comprenant un composé alcalin fort et un agent oxydant hydrosoluble et le chauffage du bain pendant une durée suffisante pour précipiter un oxyde d'argent (I, III) ou un oxyde de cobalt (II, III) interstitiellement dans l'article pour produire un article fini; et
- le retrait du bain de l'article produit.

12. Le procédé selon la revendication 11, dans lequel le composé alcalin est l'hydroxyde de sodium ou l'hydroxyde de potassium.

13. Le procédé selon la revendication 11, dans lequel l'agent oxydant est du persulfate de sodium ou du persulfate de potassium.

14. Le procédé selon la revendication 11, dans lequel l'article comprend environ 0,5 à environ 15 000 ppm du sel, exprimés par rapport au poids de l'article.

15. Le procédé selon la revendication 11, dans lequel le contact de l'article avec la première solution aqueuse comprend l'immersion de l'article dans la première solution aqueuse pendant environ 15 secondes à environ 60 secondes.

16. Le procédé selon la revendication 11, dans lequel la durée d'immersion de l'article dans la seconde solution aqueuse est d'environ 30 secondes à environ 4 minutes.

17. Le procédé selon la revendication 11, dans lequel le sel d'argent hydrosoluble est le nitrate d'argent.

18. Le procédé selon la revendication 11, dans lequel le sel de cobalt hydrosoluble est le chlorure de cobalt.

19. Le procédé selon la revendication 18, dans lequel le bain comprend en outre un fluorure hydrosoluble.

20. Le procédé selon la revendication 19, dans lequel la concentration en fluorure dans le bain est d'environ 10 à 1500 mg/l.

21. Le procédé selon la revendication 11, dans lequel la seconde solution comprend une quantité de composé alcalin suffisante pour conférer un pH d'au moins environ 13.

22. Le procédé selon la revendication 11, dans lequel le bain est chauffé à une température d'au moins 85°C.

23. Un procédé pour conférer des propriétés antimicrobiennes à un article fibreux comprenant l'imprégnation de l'article par une dispersion aqueuse de cristaux de tétroxyde de tétraargent.
